# EUROPEAN PATENT SPECIFICATION

(11) **EP 2 197 867 B1**
(45) Date of publication and mention of the grant of the patent: **02.03.2016**
(21) Application number: 08785852.8
(22) Date of filing: 05.09.2008
(51) Int. Cl.: C07D 307/46, C10L 1/02

(54) **HYDROXYMETHYLFURFURAL ETHERS FROM SUGARS OR HMF AND BRANCHED ALCOHOLS**
HYDROXYMETHYLFURFURALETHER AUS ZUCKERN ODER HMF UND VERZWEIGTEN ALKOHOLEN
ETHERS HYDROXYMÉTHYLFURFURAUX DES SUCRES OU HMF ET ALCOOLS RAMIFIÉS

(30) Priority: 07.09.2007 EP 07075773
(43) Date of publication of application: 23.06.2010
(73) Proprietor: Furanix Technologies B.V., 1014 BV Amsterdam (NL)
(72) Inventor: GRUTER, Gerardus, Johannes, Maria, 2106 BA Heemstede (NL)
(74) Representative: EP&C
(86) International application number: PCT/EP2008/007412
(87) International publication number: WO 2009/030506

(56) References cited:
- EP-A- 1 834 950
- WO-A-99/67409
- WO-A-2006/063220
- WO-A-2007/104514
- DE-A1- 3 621 517
- GARVES K: "Acid catalyzed degradation of cellulose in alcohols" JOURNAL OF WOOD CHEMISTRY AND TECHNOLOGY, MARCEL DEKKER, NEW YORK, NY, US, vol. 8, no. 1, 1988, pages 121-134, XP009067962 ISSN: 0277-3813
- COTTIER L ET AL: "Photooxygenation of 5-(hydroxymethyl)-2-furfural derivatives" BULLETIN DE LA SOCIETE CHIMIQUE DE FRANCE, SOCIETE FRANCAISE DE CHIMIE. PARIS, FR, 1986, pages 844-850, XP008086662 ISSN: 0037-8968
- DAVID W. BROWN, ET AL.: "Dehydration of Fructose in Non-aqueous Media" JOURNAL OF CHEMICAL TECHNOLOGY AND BIOTECHNOLIGY, vol. 32, 1982, pages 920-924, XP009095767
- WEIDENHAGEN R ET AL: "Ueber die Darstellung von Alkoxymethylfurfurolen und Laevulinsaeurealkylestern aus Kohlenhadraten. 2. Mitteilung" ZEITSCHRIFT WIRTSCHAFTSGRUPPE ZUCKERINDUSTRIE, vol. 85, 1935, pages 131-136, XP009095653
- TARABAN'KO V E ET AL: "Acid catalyzed conversion of carbohydrates in the presence of aliphatic alcohols at moderate temperatures" CAPLUS,, 1 January 2005 (2005-01-01), XP002487431
- HELLMUT BREDERECK: "Über isomere Dinitrophenyl-hydrazone", BERICHTE DER DEUTSCHEN CHEMISCHEN GESELLSCHAFT, vol. 65, no. 11, 7 December 1932 (1932-12-07), pages 1833-1838, XP055024224,
- GARVES K: 'Acid catalyzed degradation of cellulose in alcohols' JOURNAL OF WOOD CHEMISTRY AND TECHNOLOGY vol. 8, no. 1, 01 January 1988, MARCEL DEKKER, NEW YORK, NY, US, pages 121 - 134, XP009067962 ISSN: 0277-3813

## Description

### Technical Field

The present invention concerns the use of an ether of 5-hydroxymethylfurfural (5-(hydroxymethyl)-2-furaldehyde, or HMF) as fuel or fuel additive, and a fuel comprising such an additive.

### Background Art

Fuel, fuel additives and various chemicals used in the petrochemical industry are derived from oil, gas and coal, all finite sources. Biomass, on the other hand, is considered a renewable source. Biomass is biological material (including biodegradable wastes) which can be used for the production of fuels or for industrial production of e.g. fibres, chemicals or heat. It excludes organic material which has been transformed by geological processes into substances such as coal or petroleum.

Production of biomass derived products for non-food applications is a growing industry. Biobased fuels are an example of an application with strong growing interest.
Biomass contains sugars (hexoses and pentoses) that may be converted into value added products. Current biofuel activities from sugars are mainly directed towards the fermentation of sucrose or glucose into ethanol or via complete breakdown via Syngas to synthetic liquid fuels. EP 0641 854 describes the use of fuel compositions comprising of hydrocarbons and/or vegetable oil derivatives containing at least one glycerol ether to reduce particulate matter emissions.

More recently, the acid catalysed reaction of fructose has been re-visited, creating HMF as an intermediate of great interest. Most processes investigated have the disadvantage that HMF is not very stable at the reaction conditions required for its formation. Fast removal from the water-phase containing the sugar starting material and the acid catalyst has been viewed as a solution for this problem. Researchers at the University of Wisconsin-Madison have developed a process to make HMF from fructose. HMF can be converted into monomers for plastics, petroleum or fuel extenders, or even into fuel itself. The process by prof. James Dumesic and co-workers first dehydrates the fructose in an aqueous phase with the use of an acid catalyst (hydrochloric acid or an acidic ion-exchange resin). Salt is added to salt-out the HMF into the extracting phase. The extracting phase uses an inert organic solvent that favors extraction of HMF from the aqueous phase. The two-phase process operates at high solvent that favors extraction of HMF from the aqueous phase. The two-phase process operates at high fructose concentrations (10 to 50 wt %), achieves high yields (80% HMF selectivity at 90% fructose conversion), and delivers HMF in a separation-friendly solvent (DUMESIC, James A, et al. "Phase modifiers promote efficient production of Hydroxymethylfurfural from fructose". Science. 30 juni 2006, vol.312, no.5782, p.1933-1937). Although the HMF yields from this process are interesting, the multi-solvent process has cost-disadvantages due to the relatively complex plant design and because of the less than ideal yields when cheaper and less reactive hexoses than fructose, such as glucose or sucrose, are used as a starting material. HMF is a solid at room temperature which has to be converted in subsequent steps to useful products. Dumesic has reported an integrated hydrogenolysis process step to convert HMF into dimethylfuran (DMF), which is assumed to be an interesting gasoline additive.

In WO 2006/063220 a method is provided for converting fructose into 5-ethoxymethylfurfural (EMF) at 60 °C, using an acid catalyst either in batch during 24 hours or continuously via column elution during 17 hours. Applications of EMF were not discussed.

Also in copending patent application PCT/EP2007/002145 the manufacture of HMF ethers are described, including the use of such ethers as fuel or fuel additive. Indeed, both the methyl ether and the ethyl ether (methoxymethylfurfural, or MMF; ethoxyethylfurfural or EMF) were prepared and tested. The invention of the copending patent application, however, was limited to the use of primary aliphatic alcohols, and preferably primary C1-C5 alcohols. Use of secondary and tertiary alcohols was not considered, whereas the only example of a branched primary alcohol was considered, was a diol ("2-hydroxymethyl-propanol", which is 2-methyl-1,3-propanediol). Although MMF and EMF are useful as fuel or fuel additive, the inventors found that the ethers leave room for improvement, in particular when used in higher concentration blends with fuels such as gasoline, kerosene, diesel, biodiesel or green diesel. The inventors have therefore set out to overcome this shortfall.

Surprisingly, the inventors have found that ethers of HMF obtained from branched aliphatic alcohols have superior blending properties compared to ethers obtained from unbranched alcohol analogs. In this context, branched aliphatic alcohols are defined as
- C4-C20 primary aliphatic alcohols with branched carbon backbones
- C3-C20 secondary aliphatic alcohols with straight chain carbon backbones
- C4-C20 secondary aliphatic alcohols with branched carbon backbones
- C4-C20 tertiairy aliphatic alcohols with branched carbon backbones

The ethers of HMF with these alcohols may be produced in a reasonable yield from hexose containing feedstock, with reduced levels of by-product formation and in a manner that does not require cumbersome process measures (such as 2-phase systems) or lengthy process times.

### Disclosure of Invention

Accordingly, the current invention provides the use of a 5-hydroxymethylfurfural ether of a branched aliphatic C3-C20 monoalcohol as fuel or fuel additive. The invention also provides a fuel or fuel composition comprising such an ether.

When the reaction product of a method for the manufacture of an ether of 5-(hydroxymethyl)furfural by reacting a hexose-containing starting material with a branched aliphatic C3-C20 monoalcohol in the presence of a catalytic or sub-stoechiometric amount of an acid catalyst is used as such or when it is used as an intermediate for a subsequent conversion, the selectivity of the reaction is preferably high as the product is preferably pure. However, when the reaction product of the above method is used as a fuel, a fuel additive or as a fuel or a fuel additive intermediate, the reaction product does not necessarily need to be pure. Indeed, in the preparation of fuel and fuel additives from biomass, which in itself is a mixture of various monosaccharides, disaccharides and polysaccharides, the reaction product may contain non-interfering components such as levulinic acid derivatives and/or derivatives of pentoses and the like. For ease of reference, however, the method and the reaction product are described in terms of the reaction of a hexose-containing starting material, resulting in an ether of HMF. Also within the scope of the invention is the reaction of HMF with the branched alcohol, since HMF is believed to be produced as intermediate from the hexose-containing starting material.

The current invention also provides for the use of the reaction product made according to the above method as fuel or as fuel additive. Fuels for blending with the product of the present invention include but are not limited to gasoline and gasoline-ethanol blends, kerosene, diesel, biodiesel (refers to a non-petroleum-based diesel fuel consisting of short chain alkyl (methyl or ethyl) esters, made by transesterification of vegetable oil, which can be used (alone, or blended with conventional petrodiesel), Fischer-Tropsch liquids (for example obtained from GTL, CTL or BTL gas-to-liquids/coal-to-liquids/biomass to liquids processes), diesel-biodiesel blends and green diesel and blends of diesel and/or biodiesel with green diesel (green diesel is a hydrocarbon obtained by hydrotreating biomass derived oils, fats, greases or pyrolysis oil; see for example the UOP report OPPORTUNITIES FOR BIORENEWABLES IN OIL REFINERIES FINAL TECHNICAL REPORT, SUBMITTED TO: U.S. DEPARTMENT OF ENERGY (DOE Award Number: DE-FG36-05GO15085) The product is a premium diesel fuel containing no sulfur and having a cetane number of 90 to 100). Fuels for blending with the product of the present invention may also include one or more other furanics, wherein the expression furanics is used to include all derivatives of furan and tetrahydrofuran. The invention also provides a fuel composition comprising a fuel element as described above and the reaction product made according to the above method.

### Figures

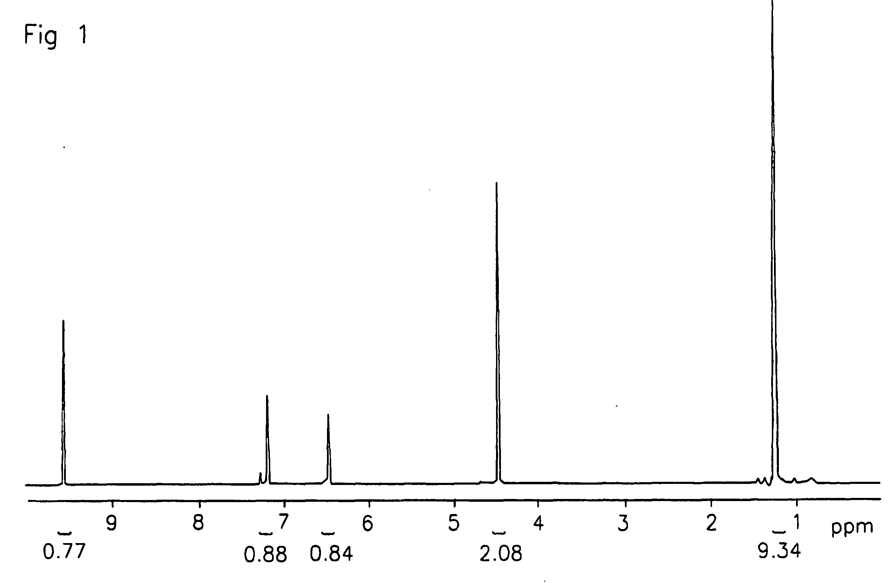

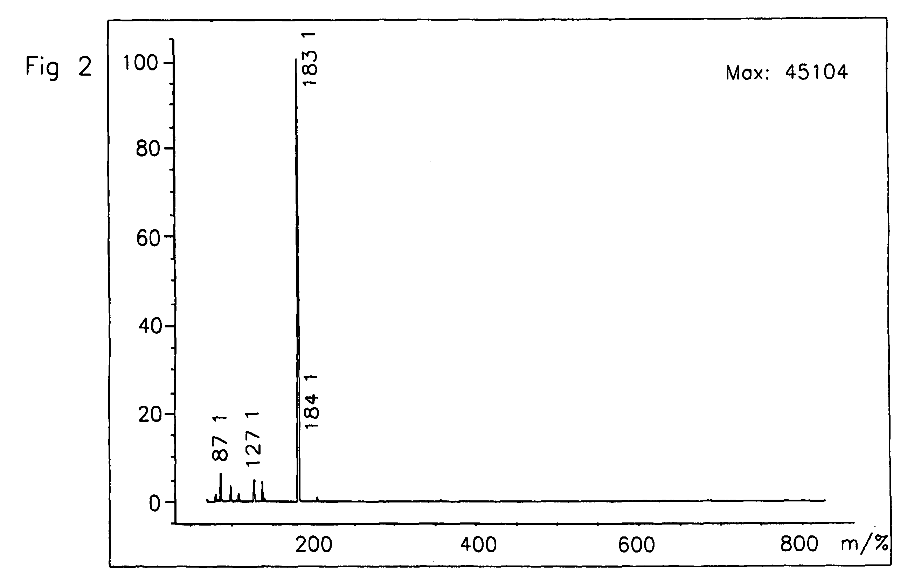

Figure 1 is the 1H-NMR of 5-(*tert*-butoxymethyl)furfural, tBMF, prepared by the process of the current invention. Figure 2 is the spectrum of tBMF using a mass spectrometer in Chemical Ionization (C.I.) Mode.

### Mode(s) for Carrying Out the Invention

Biomass resources are well known. The components of interest in biomass are the mono-, di- or polysaccharides (hereinafter referred to as hexose-containing starting material. Suitable 6-carbon monosaccharides include but are not limited to fructose, glucose, galactose, mannose, and their oxidized, reduced, etherified, esterified and amidated derivatives, e.g. aldonic acid or alditol, with glucose being the most abundant, the most economic and therefore the most preferred monosaccharide albeit less reactive than fructose. On the other hand, the current inventors have also succeeded to convert sucrose, which is also available in great abundance. Other disaccharides that may be used include maltose, cellobiose and lactose. The polysaccharides that may be used include cellulose, inulin (a polyfructan), starch (a polyglucan) and hemi-cellulose. The polysaccharides and disaccharides are converted into their monosaccharide component(s) and dehydrated during the manufacture of the 5-HMF ether.

The branched aliphatic alcohol used in the method of the current invention preferably bears a singly hydroxyl group, which may be in a primary, secondary or even tertiary position. The alcohol may comprise from 3 to 20 carbon atoms, preferably from 3 to 8 carbon atoms, whereby the alcohols with 4 or more carbon atoms have a branched carbon backbone.

In this context, branched aliphatic alcohols are defined as
- C4-C20 primary aliphatic alcohols with branched carbon backbones (R-CH2OH, with R= cyclic or non cyclic alkyl, alkenyl, and where R can contain 0, 1 or 2 elements not being C or H) such as the following non-limiting examples: 2-methylpropanol, 2,2-dimethylpropanol, 2-methylbutanol, 3-methylbutanol, 2,2-dimethylbutanol, 3,3-dimethylbutanol, 2,3-dimethylbutanol, 2-ethylbutanol, hydroxymethylcyclopentane, 2-
- hydroxymethyltetrahydrofuran, isooctanol (3-(hydroxymethyl)heptanol), etc.
- C3-C20 secondary aliphatic alcohols with straight chain carbon backbones (R-C(H)OH-R', with R, R'= n-alkyl, n-alkenyl and where R and R' can be connected to form a ring, and where R can contain 0,1 or 2 elements not being C or H) such as the following non-limiting examples: 2-propanol, 2-butanol, 2-pentanol, 3-pentanol, 4-hydroxy-1-pentene, 3-methoxy-2-propanol, etc
- C4 secondary aliphatic alcohols with branched backbones such as 2- and 3-hydroxytetrahydrofuran and C5-C20 secondary alcohols with branched carbon backbones (R-C(H)OH-R, with R, R' = alkyl, alkenyl, where R and R' can be connected to form a ring, and where R can contain 0,1 or 2 elements not being C or H) such as the following non-limiting examples: cyclopentanol, 4-cyclopentenol, 3-methyl-2-butanol, 3-methyl-2-pentanol, etc.
- C4-C20 tertiairy aliphatic alcohols with branched carbon backbones such as *tert-*Amyl alcohol (2-Methyl-2-Butanol).

Preferred alcohols used in the method of the current invention include isobutanol, *tert-*butanol, isoamyl alcohol, isooctyl alcohol. Also blends of alcohols may be used, e.g., of isobutanol and *tert*-butanol.

The amount of branched monoalcohol used during the manufacture of the HMF ether is preferably at least equimolar on the hexose content of the feedstock, but typically is used in much greater excess. Indeed, the alcohol (such as isobutanol) may be used as solvent or co-solvent. In such a case, a sufficient amount of alcohol is present to form the HMF ether.

The acid catalyst in the method of the present invention can be selected from amongst (halogenated) organic acids, inorganic acids, Lewis acids, ion exchange resins and zeolites or combinations and/or mixtures thereof. It may be a homogeneous catalyst, but heterogeneous catalysts (meaning solid) are preferred for purification reasons. The HMF ethers can be produced with a protonic, Brønsted or, alternatively, a Lewis acid or with catalysts that have more than one of these acidic functionalities.

The protonic acid may be organic or inorganic. For instance, the organic acid can be selected from amongst oxalic acid, levulinic acid, maleic acid, trifluoro acetic acid (triflic acid), methansulphonic acid or para-toluenesulphonic acid. Alternatively, the inorganic acid can be selected from amongst (poly)phosphoric acid, sulphuric acid, hydrochloric acid, hydrobromic acid, nitric acid, hydroiodic acid, optionally generated in situ.

Certain salts may be used as catalyst, wherein the salt can be any one or more of (NH₄)₂SO₄/SO₃, ammonium phosphate, pyridinium chloride, triethylamine phosphate, pyridinium salts, pyridinium phosphate, pyridinium hydrochloride/hydrobromide/perbromate, DMAP, aluminium salts, Th and Zr ions, zirconium phosphate, Sc and lanthanide ions such as Sm and Y as their acetate or trifluoroactate (triflate) salt, Cr-, Al-, Ti-, Ca-, In-ions, ZrOCl₂, VO(SO₄)₂, TiO₂, V-porphyrine, Zr-, Cr-, Ti-porphyrine.

Lewis acids selected as dehydration catalyst can be any one of ZnCl₂, AlCl₃, BF₃. Ion exchange resins can be suitable dehydration catalysts. Examples include Amberlite™ and Amberlyse™, Diaion™ and Levatit™. Other solid catalyst that may be used include natural clay minerals, zeolites, supported acids such as silica impregnated with mineral acids, heat treated charcoal, metal oxides, metal sulfides, metal salts and mixed oxides and mixtures thereof. If elevated reactions temperatures are used, as defined hereafter, then the catalyst should be stable at these temperatures.

An overview of catalysts that may be used in the method of the current invention may be found in Table 1 of the review article prepared by Mr. Lewkowski: "Synthesis, chemistry and applications of 5-hydroxymethylfurfural and its derivatives" Arkivoc. 2001, p.17-54.

A catalytic or sub-stoichiometric amount of catalyst is used. Within this range, the amount of catalyst may vary, depending on the selection of catalyst or catalyst mixture. For instance, the catalyst can be added to the reaction mixture in an amount varying from 0.01 to 40 mole % drawn on the hexose content of the biomass resource, preferably from 0.1 to 30 mole %, more preferably from 1 to 20 mole %.

In the preferred embodiment, the catalyst is a heterogeneous catalyst.

The temperature at which the reaction is performed may vary, but in general it is preferred that the reaction is carried out at a temperature from 50 to 300 degrees Celsius, preferably from 125 to 250 degrees Celsius, more preferably from 150 to 225 degrees Celsius. In general, temperatures higher than 300 are less preferred as the selectivity of the reaction reduces and as many by-products occur, inter alia caramelisation of the sugar. Performing the reaction below the lowest temperature is also less preferable because of the low reaction rate. If the reactions are carried out above the boiling temperature of water, then the reactions are preferably carried out under pressure, e.g., 10 bar nitrogen or higher.

The HMF or hexose-containing starting material is typically dissolved or suspended in a solvent, which can also be the alcohol reactant, in order to facilitate the reaction. The solvent system may be one or more selected from the group consisting of water, sulfoxides, preferably DMSO, ketones, preferably methyl ethylketone, methylisobutylketone and acetone, ethylene glycol ethers, preferably diethyleneglycol dimethyl ether (diglyme) or the reactant olefin. Also so-called ionic liquids may be used. The latter refers to a class of inert ionic compounds with a low melting point, which may therefore be used as solvent. Examples thereof include e.g., 1-H-3-methyl imidazolium chloride, discussed in "Dehydration of fructose and sucrose into 5-hydroxymethylfurfural in the presence of 1-H-3-methyl imidazolium chloride acting both as solvent and catalyst", by Claude Moreau et al; Journal of Molecular Catalysis A: Chemical 253 (2006) 165-169.

The amount of solvent is preferably present in sufficient amounts to dissolve or suspend the starting material.
The method of the current invention may be carried out in a batch process or in a continuous process, with or without recycle of (part of) the product stream to control the reaction temperature (recycle via a heat exchanger). For instance, the method of the invention can be performed in a continuous flow process. In such method, homogenous catalysts may be used and the residence time of the reactants in the flow process is between 0.1 second and 10 hours, preferably from 1 second to 1 hours, more preferably from 5 seconds to 20 minutes.

Alternatively, the continuous flow process may be a fixed bed continuous flow process or a reactive (catalytic) distillation process with a heterogeneous acid catalyst. To initiate or regenerate the heterogeneous acid catalyst or to improve performance, an inorganic or organic acid may be added to the feed of the fixed bed or reactive distillation continuous flow process. In a fixed bed process, the liquid hourly space velocity (LHSV) can be from 1 to 1000, preferably from 5 to 500, more preferably from 10 to 250 and most preferably from 25 to 100 min⁻¹.

The above process results in a stable HMF ether, which can then be used as such or be converted into a further derivative before being used as fuel and/or as fuel additive. The inventors are of the opinion that some of the products prepared by the method of the current invention are actually new. Thus, the t-butoxy-, or isooctyl-ether of HMF, prepared by using t-butanol, or isooctanol as alcohol, are new and are excellent fuel components or fuel additives. Since these alcohols may be made from biomass, this might open a class of products that are fully biomass-derived. Accordingly, these new ethers are claimed as well.

The HMF ethers of the invention can also be used as or can be converted to compounds that can be used as solvent, as monomer in a polymerization (such as 2,5-furan dicarboxylic acid or FDCA), as fine chemical or pharmaceutical intermediate, or in other applications. Oxidation of the HMF ethers using an appropriate catalyst under appropriate conditions such as for example described for p-xylene with a NHPI/Co(OAc)₂/MnOAc)₂ catalyst system in Adv. Synth. Catal. 2001, 343, 220-225 or such as described for HMF with a Pt/C catalyst system at pH < 8 in EP 0 356 703 or or such as described for HMF with a Pt/C catalyst system at pH > 7 in FR 2 669 634, all with air as an oxidant, resulted in the formation of 2,5-furan dicarboxylic acid (FDCA).

The invention concerns the use of the HMF ethers prepared by the method of the current invention as fuel and/or as fuel additive. Of particular interest is the use of the ethers in diesel, biodiesel or "green diesel", given its (much) greater solubility therein than ethanol. Conventional additives and blending agents for diesel fuel may be present in the fuel compositions of this invention in addition to the above mentioned fuel components. For example, the fuels of this invention may contain conventional quantities of conventional additives such as cetane improvers, friction modifiers, detergents, antioxidants and heat stabilizers, for example. Especially preferred diesel fuel formulations of the invention comprise diesel fuel hydrocarbons and HMF ether as above described together with peroxidic or nitrate cetane improvers such as ditertiary butyl peroxide, amyl nitrate and ethyl hexyl nitrate for example.

The addition of the HMF ether of the invention to diesel fuel results in similar NOₓ numbers and a slight increase in CO emissions; however, the addition of sufficient amounts of cetane improvers can be utilized to reduce the NOₓ and CO emissions well below the base reference fuel.

Examples are enclosed to illustrate the method of the current invention and the suitability of the products prepared therefrom as fuel. The examples are not meant to limit the scope of the invention.

The substrate conversion, the selectivity and yield were calculated according to the formulas:
Conversion = 100* [n₀ (substrate) - nₜ (substrate)] / n₀ substrate
Selectivity = 100 * nₜ (product) / [n₀ (substrate) - nₜ (substrate)]
Yield = 100 * nₜ (product) / n₀ substrate,
Where:
n₀- the initial number of moles
nₜ- the number the moles of a compound at time "t".

### Example 1. 5-(tert-Butoxymethyl)furfural (tBMF) formation from HMF and tert-butyl alcohol

To 10g (0.079 mol) of HMF was added 16.26g (0.22mol) t-butyl alcohol and 0.5g Amberlyst-15. The reactor was flushed with N₂(g) and heated to 75°C for 2 days. The mixture was concentrated and the brown oil was purified by column chromatography over SiO₂ (EtOAc : heptane, 5 : 95) to give tBMF (6.1 gr, 42.2%) as a yellow oil.

The reaction products were characterized by 1H NMR and LC-MS (CI).
See figure 1.

### Example 2. tBMF formation from fructose (or glucose) and tert-butyl alcohol

A 1.25 wt% solution of sugar (Frc or Glc) in water/tert-butanol mixture (89 or 84 wt% tert-butanol respectively) was flowed through a fixed bed (200 µl) of a Amberlyst-36 dry catalyst at 190 °C. Flow rates were selected such to achieve a space velocity of 0.25 or 0.5 min⁻¹, i.e. a contact time of 2 or 4 min.

In all cases tBMF was detected by HPLC and identified by LC-MS (CI) in the effluent stream.

### Example 3. tBMF formation from sugars and tert-butyl alcohol

The reactions were performed in the batch parallel reactors system (Block 96). In a typical experiment, 65 mg of glucose or fructose was weighted in into a reactor lined with Teflon. 0.8 ml of tert-butyl alcohol was added and the mixture reacted under nitrogen (12.5 bar) in the presence of a solid acid catalyst (6.5 mg).

| Substrate | Catalyst | T (°C) | Time (h) | Conv. (%) | s HMF (%) | s tBMF (%) |
|---|---|---|---|---|---|---|
| Fructose | CrCl₂ | 150 | 1 | 96.6 | 37.7 | 9 |
| | Zeolite HY 5 | 150 | 3 | 80.5 | 42.1 | 5.8 |
| | Zeolite HY 15 | 150 | 3 | 44 | 53 | 5.9 |
| | Amberlyst 36 Wet | 150 | 3 | 70 | 30.5 | 9.4 |
| | Amberlyst 36 Dry | 150 | 3 | 81.1 | 34.6 | 14.6 |
| Glucose | CrCl₂ | 135 | 2 | 97.8 | 21.3 | 6.5 |
| | Zeolite HY 5 | 135 | 16 | 98.9 | 14.7 | 4.4 |

### Example 4. 5-(Isopropoxymethyl) furfural (iPropMF) formation from sugars and isopropyl alcohol

In these experiments, 65 mg of glucose or fructose was weighted in into a reactor lined with Teflon. 0.8 ml of isopropyl alcohol was added and the mixture reacted under nitrogen (12.5 bar) for 1 h at 150°C, in the presence of a solid acid catalyst (6.5 mg). The two main peaks observed in the UV spectrum were identified as HMF and 5-(isopropoxymethyl)furfural (iPropMF).

| Substrate | Catalyst | Conversion [%] | HMF select. (%) | iPropMF select. (%) |
|---|---|---|---|---|
| Glucose | CrCl₂ | 97.6 | 31.1 | 10.2 |
| | Zeolite HY 5 | 68.2 | 57.7 | 1.7 |
| | Zeolite HY 15 | 71.3 | 24.8 | 22.9 |
| | Montmorillonite K 5 | 79.6 | 23.2 | 15.9 |
| Fructose | Montmorillonite K 10 | 68.4 | 22.8 | 18.6 |
| | Amberlyst 70 | 72.6 | 46.3 | 13.7 |
| | Amberlyst36Wet | 92.0 | 40.6 | 11.3 |
| | SulphatedZirconia | 22.7 | 9.6 | 0.0 |
| | Amberlyst36Dry | 98.7 | 33.5 | 14.5 |

### Example 5- 5-(tert-butoxymethyl) furfural (tBuMF) formation from HMF and tert-butyl alcohol

In these experiments, a mixture of 0.36 mmol of HMF, and 0.8 ml of tert-butyl alcohol reacted under nitrogen (12.5 bar), in the presence of a solid acid catalyst (6.5 mg), 3 h at 100°C.

| Catalyst | Conv. (%) | s (tBuMF) (%) |
|---|---|---|
| Montmorillonite K5 | 59.3 | 79.1 |
| Zeolite HY 5 | 46.2 | 76.8 |
| Al(III) triflate | 51.2 | 78.6 |
| Zeolite HY 15 | 49.3 | 76.9 |

### Example 6. diesel fuel applications

### Fuel solubility

Fuel solubility is a primary concern for diesel fuel applications. Not all highly polar oxygenates have good solubility in the current commercial diesel fuels. Results show that in the 5 vol%, in the 25 vol% and in the 40 vol% blends of tBMF with commercial diesel, both liquid blend components are completely miscible. In a comparative set of experiments it was shown that ethoxymethylfurfural (EMF) is completely miscible in a 5 vol% blend with commercial diesel, but that phase separation occurs with the 25 vol% and with the 40 vol% blends of EMF and diesel.

### Cetane number

Oxygenated fuel additives may reduce the natural cetane number of the base diesel fuel. A 0.1 vol% blend of tBMF with additive free diesel fuel was prepared at an outside laboratory for cetane determination according to an ASTM D 6890 certified method. While the reference additive-free diesel showed an average cetane number of 52.5, surprisingly the 0.1 vol% tBMF blend showed an increase with 0.5 to an average cetane number of 53.0.

### Oxidation stability

Likewise, oxygenated fuel additives, certainly when containing an aldehyde functional group, often reduce the oxidation stability of the base diesel fuel. A 0.1 vol% blend of tBMF with additive free diesel fuel was prepared at an outside laboratory for oxidation stability determination according to NF en ISO 12205 certified methods. Surprisingly, both the reference additive-free diesel and the 0.1 vol% tBMF blend showed the same oxidation stability, indicating that the oxygenated tMBF added to an additive free diesel base fuel does not decrease the oxidation stability of the blend relative to the pure base diesel.

### Example 7. Emission engine testing

In a D9B diesel engine of a Citroen Berlingo test car, comparative testing is performed with normal commercial diesel as a fuel and the same commercial diesel to which 25 vol. % 5-(t-butoxymethyl)furfural (tBMF) was added, respectively. tBMF is added as a liquid and does not yield any mixing or flocculation problems up to a 40 vol% blend ratio. The engine is run stationary with regular diesel initially, after which the fuel supply is switched to the 40 vol% tBMF-diesel blend.

During stationary operation with the commercial diesel fuel and with the 25 vol% tBMF blend, the following measurements were made: total particulate matter, volume, O₂. CO, CO₂, NOₓ (NO + NO₂) and total hydrocarbons.
Total particulate matter was sampled according to NEN-EN 13284-1
Particle size distribution was sampled according to VDI 2066-5
Volume was measured according to ISO 10780
Gases were sampled according to ISO 10396
O₂, CO and CO₂ were analysed according to NEN-ISO 12039
NOₓ (NO + NO₂) was analysed according to NEN-ISO 10849
Total hydrocarbons were analysed according to NEN-EN 13526.

**Table 1, gas analysis results of 100% commercial diesel fuel.**

| Experiment | Component | Average Concentration | Emission |
|---|---|---|---|
| 1 | CO | 191 mg/Nm³ | 13 g/h |
| | CO₂ | 2.4 % v/v | - |
| | O₂ | 17.8% v/v | - |
| | TOC (C₃H₈) | 29 mg/Nm³ | 2 g/h |
| | NOₓ | 323 mg/Nm³ | 21 g/h |

**Table 2, particulate matter results of 100% commercial diesel fuel.**

| Experiment | Volume | | Total particulate matter | | Particle size |
|---|---|---|---|---|---|
| | Actual [m3/h] | Normal [Nm3/h] | Concentration [mg/Nm3] | Emission [g/h] | PSD <10 µm [%] |
| 1 | 80 | 60 | 6.1 | | 98,5 |

**Table 3, gas analysis results of blend of commercial diesel with 25 vol% tBMF.**

| Experiment | Component | Average Concentration | Emission |
|---|---|---|---|
| 2 | CO | 243 mg/Nm³ | 16 g/h |
| | CO₂ | 2.5 % v/v | - |
| | O₂ | 17.7% v/v | - |
| | TOC (C₃H₈) | 40 mg/Nm³ | 3 g/h |
| | NOₓ | 333 mg/Nm³ | 22 g/h |

**Table 4, particulate matter results of blend of commercial diesel with 25 vol% tBMF.**

| Experiment | Volume | | Total particulate matter | | Particle |
|---|---|---|---|---|---|
| | Actual | Normal [Nm3/h] | Concentration [mg/Nm3] | Emission [g/h] | PSD <10 µm [%] |
| 3b (**) | 80 | 60 | 5.1 | | 100 |

### References

- DUMESIC, James A, et al. "Phase modifiers promote efficient production of Hydroxymethylfurfural from fructose". Science. 30 June 2006, vol.312, no.5782, p.1933-1937.
- WO 2006/063220
- Chapter 15 of Advanced Organic Chemistry, by Jerry March, and in particular under reaction 5-4. (3rd ed., © 1985 by John Wiley & Sons, pp. 684-685).
- LEWKOWSKI, Jaroslaw. Synthesis, chemistry and applications of 5-hydroxymethylfurfural and its derivatives. Arkivoc. 2001, p.17-54.
- MOREAU, Claude, et al. "Dehydration of fructose and sucrose into 5-hydroxymethylfurfural in the presence of 1-H-3-methyl imidazolium chloride acting both as solvent and catalyst", Journal of Molecular Catalysis A: Chemical 253 (2006) p. 165-169.
- EP 0641 854
- UOP report OPPORTUNITIES FOR BIORENEWABLES IN OIL REFINERIES FINAL TECHNICAL REPORT, SUBMITTED TO: U.S. DEPARTMENT OF ENERGY (DOE Award Number: DE-FG36-05GO15085))
- Adv. Synth. Catal. 2001, 343, 220-225
- EP 0 356 703
- FR 2 669 634

## Claims

1. Use of a 5-(hydroxymethyl)furfural ether of a branched aliphatic C₃-C₂₀ monoalcohol as fuel or fuel additive.

2. A fuel or fuel composition comprising a 5-(hydroxymethyl)furfural ether of a branched aliphatic C₃-C₂₀ monoalcohol, blended with one or more of gasoline and gasoline-ethanol blends, kerosene, diesel, biodiesel (a non-petroleum-based diesel fuel consisting of short chain alkyl (methyl or ethyl) esters, made by transesterification of vegetable oil), Fischer-Tropsch liquids, diesel-biodiesel blends and green diesel (a hydrocarbon obtained by hydrotreating biomass derived oils, fats, greases or pyrolysis oil; containing no sulfur and having a cetane number of 90 to 100) and blends of diesel and/or biodiesel with green diesel and other derivatives of furan or tetrahydrofuran.

3. Fuel or fuel composition according to claim 2, wherein the ether is the reaction product of a method for the manufacture of an ether of 5-(hydroxymethyl)furfural by reacting a hexose-containing starting material with a branched aliphatic C₃ - C₂₀ monoalcohol in the presence of a catalytic or sub-stoichiometric amount of an acid catalyst.

4. Ether of 5-(hydroxymethyl)furfural, which is 5-(hydroxymethyl)furfural iso-octyl ether, or 5-(hydroxymethyl)furfural tert-butyl ether.

## Patentansprüche

1. Verwendung eines 5-(Hydroxymethyl)furfuralethers von einem verzweigten aliphatischen C₃-C₂₀-Monoalkohol als Kraftstoff oder Kraftstoffadditiv.

2. Kraftstoff oder Kraftstoffzusammensetzung umfassend einen 5-(Hydroxymethyl)-furfuralether von einem verzweigten aliphatischen C₃-C₂₀-Monoalkohol, vermischt mit einem oder mehreren von Benzin und Benzin-Ethanol-Mischungen, Kerosin, Diesel, Biodiesel (ein nicht auf Erdöl basierter Dieselkraftstoff, der aus kurzkettigen Alkylestern (Methyl-oder Ethylestern) besteht und durch Umesterung von Pflanzenöl hergestellt wird), Fischer-Tropsch-Flüssigkeiten, Diesel-Biodiesel-Mischungen und Gründiesel (ein Kohlenwasserstoff, der durch Hydrotreating von aus Biomasse gewonnenen Ölen, Fetten, Schmierfetten oder Pyrolyseöl erhalten wird; keinen Schwefel enthält und eine Cetanzahl von 90 bis 100 aufweist) und Mischungen von Diesel und/oder Biodiesel mit Gründiesel und anderen Derivaten von Furan oder Tetrahydrofuran.

3. Kraftstoff oder Kraftstoffzusammensetzung nach Anspruch 2, wobei der Ether das Reaktionsprodukt von einem Verfahren zur Herstellung eines Ethers von 5-(Hydroxymethyl)furfural durch Umsetzen eines Hexose enthaltenden Ausgangsmaterials mit einem verzweigten aliphatischen C₃-C₂₀-Monoalkohol in Gegenwart einer katalytischen oder unterstöchiometrischen Menge eines Säurekatalysators ist.

4. Ether von 5-(Hydroxymethyl)furfural, welcher 5-(Hydroxymethyl)furfural-iso-octylether oder 5-(Hydroxymethyl)furfural-tert-butylether ist.

## Revendications

1. Utilisation d'un éther de 5-(hydroxyméthyl)furfural d'un monoalcool en C₃ à C₂₀ aliphatique ramifié en tant que carburant ou additif pour carburant.

2. Carburant ou composition de carburant comprenant un éther de 5-(hydroxyméthyl)furfural d'un monoalcool en C₃ à C₂₀ aliphatique ramifié, mélangé à un ou plusieurs éléments choisis parmi les essence et mélange d'essence-éthanol, kérosène, diesel, biodiesel (un carburant diesel qui n'est pas à base de pétrole se composant d'esters d'alkyle de courte chaîne (méthyle ou éthyle), préparé par transestérification d'une huile végétale) liquides de Fischer-Tropsch, mélanges de diesel-biodiesel et diesel vert (un hydrocarbure obtenu par hydrotraitement d'huiles, matières grasses, graisses dérivées d'une biomasse ou huile de pyrolyse ; ne contenant pas de soufre et ayant un indice de cétane de 90 à 100) et des mélanges de diesel et/ou biodiesel avec un diesel vert et d'autres dérivés du furane ou du tétrahydrofurane.

3. Carburant ou composition de carburant selon la revendication 2, dans lequel/laquelle l'éther est le produit réactionnel d'un procédé de préparation d'un éther de 5-(hydroxyméthyl)furfural en faisant réagir une matière première contenant de l'hexose avec un monoalcool en C₃ à C₂₀ aliphatique ramifié en présence d'une quantité catalytique ou substcechiométrique d'un catalyseur acide.

4. Éther de 5-(hydroxyméthyl)furfural, qui est l'éther isooctylique de 5-(hydroxyméthyl)furfural ou l'éther tert-butylique de 5-(hydroxyméthyl)furfural.
